# EUROPÄISCHE PATENTSCHRIFT

(11) **EP 3 488 202 B1**
(45) Veröffentlichungstag und Bekanntmachung des Hinweises auf die Patenterteilung: **25.08.2021**
(21) Anmeldenummer: 17748397.1
(22) Anmeldetag: 20.07.2017
(51) Int. Cl.: G01G 17/06, A61M 1/00, A61M 1/28

(54) **VORRICHTUNG UND EIN VERFAHREN ZUM AUTOMATISCHEN, GEWICHTSABHÄNGIGEN FÜLLEN EINES SCHLAUCHSYSTEMS**
DEVICE AND A METHOD FOR AUTOMATIC WEIGHT-DEPENDENT FILLING OF A HOSE SYSTEM
DISPOSITIF ET PROCÉDÉ DE REMPLISSAGE AUTOMATIQUE D'UN SYSTÈME DE TUYAUX EN FONCTION DU POIDS

(30) Priorität: 20.07.2016 DE 102016008888
(43) Veröffentlichungstag der Anmeldung: 29.05.2019
(60) Teilanmeldung: 21173618.6
(73) Patentinhaber: Fresenius Medical Care Deutschland GmbH, 61352 Bad Homburg (DE)
(72) Erfinder: HASSLER, Manuel, 60433 Frankfurt am Main (DE); WOLF, Klaus, 97450 Arnstein (DE); WABEL, Peter, 64288 Darmstadt (DE)
(74) Vertreter: Herrmann, Uwe
(86) Internationale Anmeldenummer: PCT/EP2017/000887
(87) Internationale Veröffentlichungsnummer: WO 2018/015020

(56) Entgegenhaltungen:
- WO-A2-2013/067359
- US-A- 5 445 610
- US-A1- 2007 276 328

## Beschreibung

Die Erfindung betrifft eine Vorrichtung und ein Verfahren zum automatischen, gewichtsabhängigen Füllen eines Schlauchsystems, insbesondere für einen gravimetrischen Cycler für die Peritonealdialyse mit nur einer Waage, wobei das Schlauchsystem wenigstens drei Leitungsabschnitte umfasst, welche zum Verbinden des Schlauchsystems mit wenigstens einem Drainagebeutel, wenigstens einem Lösungsbeutel und einem Patienten ausgebildet sind.

Vor dem Beginn von Dialysebehandlungen ist es besonders wichtig, dass die Leitungen dabei verwendeter Schlauchsysteme luftfrei und gefüllt sind. Dies wird durch das Spülen bzw. Füllen des Schlauchs bzw. der Schlauchsysteme mit Dialyseflüssigkeit erreicht. Hierzu soll möglichst nur so viel Dialyselösung aufgewendet werden, wie notwendig ist, um die Luft aus dem System zu Spülen und den Schlauch mit Flüssigkeit zu befüllen. Da sowohl die Dialysatbehältnisse als auch die Dialyselösung in der Regel transparent sind und die Flüssigkeit verhältnismäßig schnell durch das Schlauchsystem strömt, kann dies zur sicheren Anwendung und effizienten Nutzung der Dialyselösung für den Spülprozess nur unter Einhaltung einer sehr kurzen Reaktionszeit durchgeführt werden.

Die Begriffe "Spülen" und "Füllen" stellen im Rahmen der Erfindung Synonyme dar.

Aus dem Stand der Technik sind Vorrichtungen bzw. Verfahren bekannt, welche bspw. mit einem Zentralprozessor arbeiten, welcher mit einer Wägeeinrichtung und einer Uhr verbunden ist um die Füll-, Dwell- und Drainzeit verfolgen zu können. Dabei sind Systeme mit nur einer Waage bekannt. Allerdings finden Füllphase und Drainphase niemals zur gleichen Zeit statt, so dass die Verwendung einer einzelnen Wägeeinrichtung ausreichend ist. Spülprozesse, die sich automatisch steuern lassen und über eine Gewichtsbilanzierung mit nur einer Waage erfasst werden können sind dabei unbekannt.

Beispielsweise offenbart die US2007/0276328A1 ein automatisches Dialysesystem, bei dem ein Patient mittels Vakuum drainiert wird. Zudem ist aus der US5445610 ein portabler Cycler für die Peritonealdialyse bekannt. Weiterer Stand der Technik ist die Druckschrift WO2013/067359A2.

Aus dem Stand der Technik sind keine Vorrichtungen oder Verfahren bekannt, die für das Spülen des Schlauchsets, insbesondere bei gravimetrisch arbeitenden Geräten in der Peritonealdialyse mit einer Kennlinie arbeitet, welche gewichtsabhängig die Öffnungszeit der Ventile in Verbindung mit nur einer Waage oder Wägezelle steuert. Bei der erfindungsgemäßen Vorrichtung wird die sich verändernde Gewichtsabnahme während des Fluidablaufs beim Spülprozess berücksichtigt, was ebenfalls nicht aus dem Stand der Technik bekannt ist. Auch sind aus dem Stand der Technik keine Vorrichtungen oder Verfahren bekannt, die eine Erkennung der ordnungsgemäßen Öffnung der Beutel- bzw. Schlauchklemmen erlauben.

Der Begriff "Beutel" steht im Rahmen der vorliegenden Erfindung stellvertretend für beliebige Behältnisse, unabhängig davon, ob sie starre und/oder flexible Wandungen aufweisen.

Vor diesem Hintergrund ist es Aufgabe der Erfindung eine verbesserte Vorrichtung bzw. ein verbessertes Verfahren zum Spülen eines Schlauchsystems bereitzustellen, bei dem ein vollständiges Befüllen mit Dialyseflüssigkeit gewährleistet und dabei die Dialysatmenge besser bzw. exakt portionierbar ist. Hierdurch soll nur die tatsächlich benötigte Menge an Dialysat zum Spülen der Schlauchleitungen aufgewendet werden. Eine weitere Aufgabe der Erfindung besteht darin, ein ordnungsgemäßes Überprüfen von Dialysatbeutelkonnektoren und/oder Schlauchklemmen bzw. von entsprechenden Ventilen zu ermöglichen, wodurch die Patientensicherheit und der Bedienkomfort gesteigert werden.

Diese Aufgabe wird erfindungsgemäß durch ein Verfahren mit den Merkmalen des Anspruchs 1 gelöst. Vorteilhafte Ausbildungen sind Gegenstand der Ansprüche. Demnach ist ein Verfahren zum automatischen, gewichtsabhängigen Spülen bzw. Füllen eines Schlauchsystems, insbesondere für einen gravimetrischen Cycler für die Peritonealdialyse mit nur einer Waage vorgesehen, wobei das Schlauchsystem wenigstens drei Leitungsabschnitte zum Verbinden mit wenigstens einem Drainagebeutel, wenigstens einem Lösungsbeutel und einem Patienten umfasst, wobei ferner wenigstens ein Beutelventil und wenigstens ein Drainageventil geöffnet werden und eine erste Öffnungsdauer der Ventile aus dem mittels der Waage gemessenen Gewichts des Lösungsbeutels und aus einer Kennlinie abgeleitet wird.

Der Begriff des Lösungsbeutels kann dabei einen einzelnen oder eine Mehrzahl von Lösungsbeuteln umfassen. Die aus der Kennlinie ableitbare Öffnungsdauer kann sich dabei auf die Öffnungsdauer eines oder beider Ventile beziehen. Denkbar ist auch, dass abweichende Öffnungsdauern zu den einzelnen Ventilen hinterlegt sind. Mit den Ventilen gemäß Anspruch 1 können das Beutelventil und das Drainageventil gemeint sein. Verfahrensgemäß kann eine Regelung/Steuerung vorliegen, die mit der Waage gekoppelt sein kann und den Lösungsbeutel bzw. dessen Gewicht erfassen kann. Ferner kann verfahrensgemäß auf die Kennlinie zurückgegriffen werden, welche bspw. in der Regelung/Steuerung implementiert bzw. abgespeichert sein kann. Die Regelung/Steuerung kann so das gemessene Gewicht des Lösungsbeutels mit der hinterlegten Kennlinie abgleichen und aus der Kennlinie eine erste Öffnungsdauer ermitteln, für die die Ventile geöffnet werden sollen. Das Gewicht des Lösungsbeutels ermöglicht es dabei, einen guten Näherungswert für die Zeit zu finden, die erforderlich ist, um das Schlauchsystem mit Dialysatlösung zu füllen.

Dabei ist es auch denkbar, dass der Luftdruck und/oder die Gerätehöhe bei der Erstellung der Kennlinie berücksichtigt werden.

In einer bevorzugten Ausführung ist denkbar, dass mittels der Waage erfasst wird, ob die Ventile geöffnet worden sind. Um festzustellen, ob trotz eines entsprechenden Öffnungssignals an die Ventile bspw. eine Fehlfunktion wenigstens eines der Ventile vorliegt, kann mittels der oben erwähnten Regelung/Steuerung während der Öffnungsphase der Ventile das Gewicht des Lösungsbeutels weiterhin erfasst werden. Falls dabei trotz ausgegebenen Signals zum Öffnen der Ventile keine Reduzierung des Gewichts des Lösungsbeutels erfasst wird, kann dies als Hinweis auf eine Fehlfunktion von wenigstens einem der Ventile interpretiert oder auf ein nicht korrekt aufgebautes System geschlossen werden und von der Regelung/Steuerung entsprechend erfasst bzw. angezeigt werden. Hierzu kann verfahrensgemäß ein Hinweis an eine Bedienperson über bspw. eine Anzeige oder Leuchtdiode oder ein akustisches Signal ausgegeben werden.

In einer weiteren, bevorzugten Ausführung ist denkbar, dass eine Gewichtsabnahme des Lösungsbeutels während der Öffnungsdauer der Ventile mittels der Waage erfasst wird und in Abhängigkeit von der erfassten Gewichtsabnahme die erste Öffnungsdauer modifiziert wird. Hierdurch wird eine zweite, korrigierte Öffnungsdauer errechnet. Wird während der Öffnungsdauer der Ventile bspw. festgestellt, dass das Gewicht des Lösungsbeutels zu langsam oder zu schnell fällt, so kann die erste Öffnungsdauer der Ventile entsprechend verlängert oder verkürzt werden. Hierbei kann berücksichtigt werden, ob die Gewichtsabnahme des Lösungsbeutels einer hinterlegten Soll-Gewichtsabnahme nahekommt bzw. diese erreicht. Ist dies nicht der Fall, so kann die Öffnungsdauer entsprechend so modifiziert bzw. verlängert werden, dass die Soll-Gewichtsabnahme erreicht wird. Umgekehrt kann bei Feststellen einer schnellen Gewichtsabnahme bzw. einer schnellen Annäherung an eine Soll-Gewichtsabnahme die erste Öffnungsdauer so verkürzt werden, dass möglichst keine größere Gewichtsabnahme als die Soll-Gewichtsabnahme auftritt. Die zweite Öffnungsdauer kann somit auch kürzer ausfallen als die erste Öffnungsdauer.

In einer weiteren bevorzugten Ausführung ist ferner denkbar, dass nach Schließen der Ventile eine Aufforderung an eine Bedienperson ergeht, eine Schlauchklemme zu öffnen und hierdurch Dialyseflüssigkeit in Richtung eines Konnektors geleitet wird. Die Schlauchklemme kann hierbei ein drittes auf das Schlauchsystem wirkende Ventil sein und bspw. manuell von der Bedienperson bedienbar ausgebildet sein. Durch das Öffnen der Schlauchklemme ist es möglich das Schlauchsystem mit einem Patientenzugang bzw. mit einer Patientenleitung zu verbinden. Bei der Bedienperson kann es sich insbesondere um einen Patienten handeln, der eine Peritonealdialyse vorbereitet.

Ferner kann in einer weiteren, bevorzugten Ausführung vorgesehen sein, dass mittels der Waage erfasst wird, ob die Schlauchklemme geöffnet worden ist. Wurde bspw. eine Aufforderung an eine Bedienperson ausgegeben, die Schlauchklemme zu öffnen und wird im Anschluss daran keine Verringerung des Gewichts des Lösungsbeutels erfasst, so kann dies verfahrensgemäß als Hinweis gedeutet werden, dass die Schlauchklemme weiterhin geschlossen ist. Demnach kann ein entsprechender Hinweis an die Bedienperson bzw. eine entsprechende Warnmeldung ausgegeben werden, die Schlauchklemme zum Durchspülen des entsprechenden Schlauchabschnitts zu öffnen.

In einer weiteren Ausführungsform kann auch ein gewichtsabhängiger Nachfüllprozess vorgesehen sein. Für ein solches Nachfüllen ist dann eine geringere Zeitspanne für das Öffnen der Ventile vorgesehen.

Das erfindungsgemäße Verfahren, insbesondere das Spülen bzw. Füllen des Schlauchsystems wird durchgeführt, bevor der Patient an das Schlauchsystem angeschlossen ist. Es findet somit zunächst das Spülen bzw. Füllen des Schlauchsystems statt. Ist dieses abgeschlossen, wird der Patient konnektiert und die Behandlung kann beginnen.

Die Erfindung ist ferner auf eine Vorrichtung zum automatischen, gewichtsabhängigen Füllen eines Schlauchsystems nach Anspruch 7 gerichtet, insbesondere für einen gravimetrischen Cycler für die Peritonealdialyse mit nur einer Waage gerichtet, wobei das Schlauchsystem wenigstens drei Leitungsabschnitte zum Verbinden mit wenigstens einem Drainagebeutel, wenigstens einem Lösungsbeutel und einem Patienten umfasst, wobei die Vorrichtung eine Regelung/Steuerung umfasst, in der wenigstens eine Kennlinie hinterlegt ist, die Ventil-Öffnungsdauern in Abhängigkeit von dem Gewicht eines Lösungsbeutels angibt. Bei dem Lösungsbeutel kann es sich um einen Behälter für eine Dialysatlösung handeln und die Ventil-Öffnungsdauern können für beide Ventile gleich oder unterschiedlich sein.

Die Vorrichtung kann dabei in einer bevorzugten Ausführung zum Öffnen von wenigstens einem Beutelventil und/oder Drainageventil in Abhängigkeit von dem Gewicht des Lösungsbeuels eingerichtet sein. Das Öffnen der Ventile kann hierbei automatisch erfolgen, wobei die Ventile entsprechend über die Regelung/Steuerung ansteuerbar sind.

In einer weiteren Ausführung ist ferner denkbar, dass die Regelung/Steuerung dazu ausgelegt ist, anhand einer erfassten Gewichtsänderung das korrekte Öffnen des Beutelventils und/oder Drainageventils zu verifizieren. Ein korrektes Öffnen der Ventile liegt dann vor, wenn auf ein entsprechendes Signal der Regelung/Steuerung die Ventile auch geöffnet worden sind. Dies kann dadurch festgestellt werden, dass beim Vorliegen eines korrekten Öffnens eine entsprechende Gewichtsreduktion des Lösungsbeutels erfassbar ist. Ist dies nicht der Fall, so ist dies als Hinweis auf ein nicht korrektes Öffnen der Ventile zu deuten und ein entsprechendes Warnsignal kann ausgegeben werden. Umgekehrt kann bei korrektem Öffnen der Ventile ein Bestätigungssignal ausgegeben werden. Hierdurch erfolgt die entsprechende Verifikation.

In einer weiteren bevorzugten Ausführung kann vorgesehen sein, dass die Vorrichtung eine Ausgabeeinrichtung umfasst, die zum Ausgeben von Informationen an eine Bedienperson ausgebildet ist. Die Ausgabeeinrichtung kann eine Display, eine Leuchte und/oder eine akustische Ausgabeeinrichtung umfassen, die jegliche von der Vorrichtung erfassten Parameter oder ermittelten Informationen entsprechend an eine Bedienperson ausgeben können.

Es kann auch vorgesehen sein, dass die Regelung/Steuerung dazu ausgelegt ist, anhand einer erfassten Gewichtsänderung das korrekte Öffnen eines weiteren Ventils, insbesondere einer manuell bedienbaren Schlauchklemme zu verifizieren. Wurde bspw. ein Signal an eine Bedienperson gegeben, eine Schlauchklemme zu öffnen und wird von der Regelung/Steuerung eine Gewichtsabnahme des Lösungsbeutels erfasst, so kann dies von der Vorrichtung als Hinweis darauf gedeutet werden, dass die Schlauchklemme tatsächlich geöffnet worden ist, wodurch die Verifizierung der Öffnung erfolgt.

Weitere Einzelheiten und Vorteile der Erfindung sind anhand der Figuren aufgezeigt. Dabei zeigen:
- Fig. 1:: eine perspektivische Ansicht einer erfindungsgemäßen Vorrichtung;
- Fig. 2:: eine Ansicht eines Schlauchsystems;
- Fig. 3:: eine beispielhaft gezeigte Kennlinie und
- Fig. 4:: eine beispielhaft gezeigte linearisierte Kennlinie.

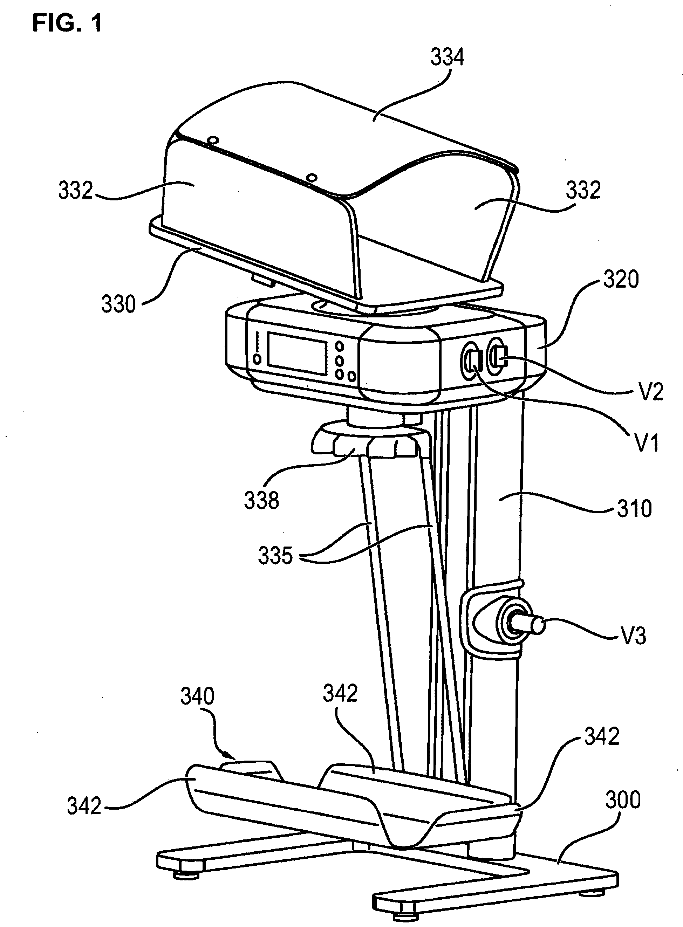

Figur 1 zeigt ein Peritonealdialysegerät gemäß der vorliegenden Erfindung in einer perspektivischen Ansicht.

Das Peritonealdialysegerät weist einen U-förmigen Standfuß 300 auf, von dem sich nach vertikal oben ein Gerätegehäuseträger 310 erstreckt. An dessen oberen Ende befindet sich das Gerätegehäuse 320. In dem Gerätegehäuse 320 befindet sich die zum Betrieb des Gerätes erforderliche Elektronik, wie z.B. Steuer- und Regelungseinheiten, sowie die Bedien- und/oder Anzeigeeinheiten.

Unmittelbar oberhalb des Gerätegehäuses 320 ist direkt mit diesem verbunden die Heizschale 330 angeordnet, die zur Aufnahme von Lösungsbeuteln dient, die frisches, dem Patienten zuzuführendes Dialysat enthalten.

Unten an dem Gerätegehäuse 320 bzw. an der Wägezelle 338 befindet sich ein Gestänge 335, an dem die Wägeschale 340 angeordnet ist. Die Wägeschale 340 dient zur Aufnahme eines oder mehrerer Aufnahmebeutel, in die das gebrauchte, vom Patienten stammende Dialysat gelangt.

Wie dies aus Figur 1 ersichtlich ist, befindet sich die Wägeschale 340 unmittelbar über dem Fußboden, auf dem das Peritonealdialysegerät mit seinem Standfuß 300 aufsteht. Aus Figur 1 geht weiter hervor, dass der Standfuß ein flaches Profil aufweist, so dass die Wägeschale 340 weit unten angeordnet werden kann.

Der Abstand zwischen dem Boden der Wägeschale 340 und dem Fußboden beträgt beispielsweise wenige cm, z.B. 5 cm bis 10 cm und vorzugsweise 7,5 cm bis 8,5 cm.

Der Abstand zwischen dem Boden der Heizschale 330 und dem Boden der Wägeschale 340 liegt vorzugsweise zwischen 80 cm und 1,2 m.

Die Fluidsteuerung zum und vom Patienten erfolgt über Ventile, wobei das oder die Ventile für die Fluidverbindung zwischen dem bzw. den Lösungsbeuteln, die sich in der Heizschale 330 befinden, und dem Patienten am Gerätgehäuse 320 angeordnet sind. Diese Ventile sind in Figur 1 mit dem Bezugszeichen V1, V2 gekennzeichnet. Das Drainageventil V3, das zwischen dem Patienten und dem Aufnahmebeutel für verbrauchtes Dialysat angeordnet ist, befindet sich an dem Gerätegehäuseträger 310. Es ist in einer Höhe von ca. 40 cm bis 50 cm und vorzugsweise von 45 cm über dem Boden angeordnet. Dies erlaubt eine bequeme Bedienung durch den Patienten.

Wie aus Figur 1 ersichtlich, erstreckt sich das Gestänge 335 von der Rückseite der Wägeschale 340 nach oben zu der Unterseite des Gerätegehäuses 320. Somit ist eine gute Zugänglichkeit von der Vorderseite zu der Wägeschale 340 gegeben.

Das Gestänge 335 ist an einer Wägezelle 338 angeordnet, die sich an oder in dem Gerätegehäuse 320 befindet.

Sowohl die Heizschale 330 als auch die Wägeschale 340 weisen eine Auflagefläche für den oder die Beutel auf, die den Boden darstellt. Ausgehend von dem Boden erstrecken sich Seitenwände 332 und 342 nach oben. Diese haben die Aufgabe, die jeweils aufgenommenen Beutel sicher in der Schale zu halten. Dies ist insbesondere dann von Bedeutung, wenn in der Heizschale 330 und in der Wägeschale 340 eine Mehrzahl von Beuteln aufgenommen ist.

Die Seitenwände 332 und 342 können durch eine Steckverbindung befestigt sein oder relativ zu der Auflagefläche verschwenkbar sein, so dass die Beutel leicht eingelegt und entnommen werden können.

Die Heizschale 330 ist darüber hinaus mit einer oberen Abdeckung 334 versehen, die die Aufgabe hat, die Wärme möglichst im Bereich der Lösungsbeutel zu halten, die sich in der Heizschale 330 befinden.

Das Schlauchsystem kann erfindungsgemäß so angeordnet sein, dass es von der Wägeschale 340 bzw. von der Wage nicht gewogen wird. Hierzu kann es an Strukturen befestigt sein, die vom Peritonealdialysegerät getrennt sind, so dass die Gewichtskraft des Schlauchsystems nicht auf die Waage wirkt.

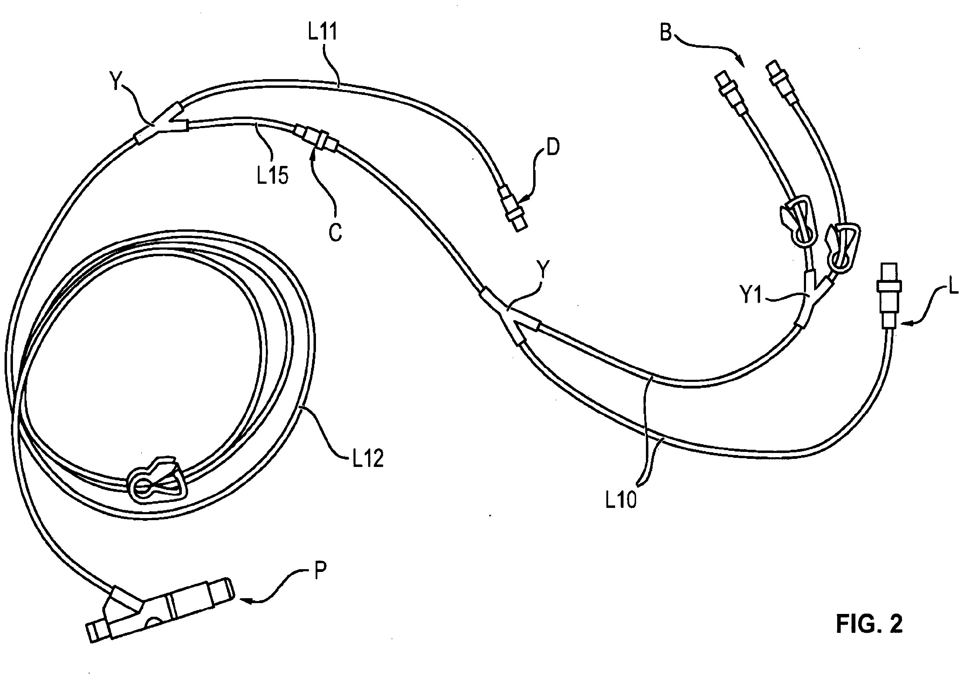

Wie dies aus Figur 2 ersichtlich ist, stehen die Leitungen, die mit den Konnektoren B in Verbindung stehen, über ein Y-Stück Y1 in Verbindung. Die aus diesem mündende gemeinsame Leitung steht über das weitere Y-Stück Y2 mit der Leitung in Verbindung, die zu dem Last-Bag führt.

Die Länge des Leitungsabschnittes zwischen dem Konnektor C und dem Y-Stück "Y2" liegt vorzugweise im Bereich von 3 cm bis 7 cm, die Länge des Leitungsabschnittes zwischen dem Y-Stück "Y2" und dem Y-Stück "Y1" liegt vorzugsweise im Bereich von 15 cm bis 25 cm und, die Länge des Leitungsabschnittes zwischen dem Y-Stück "Y2" und dem Konnektor L liegt vorzugsweise im Bereich zwischen 40 cm und 50 cm und die Länge des Leitungsabschnittes zwischen dem Y-Stück "Y1" und den Konnektoren B liegt vorzugsweise im Bereich zwischen 15 cm bis 25 cm.

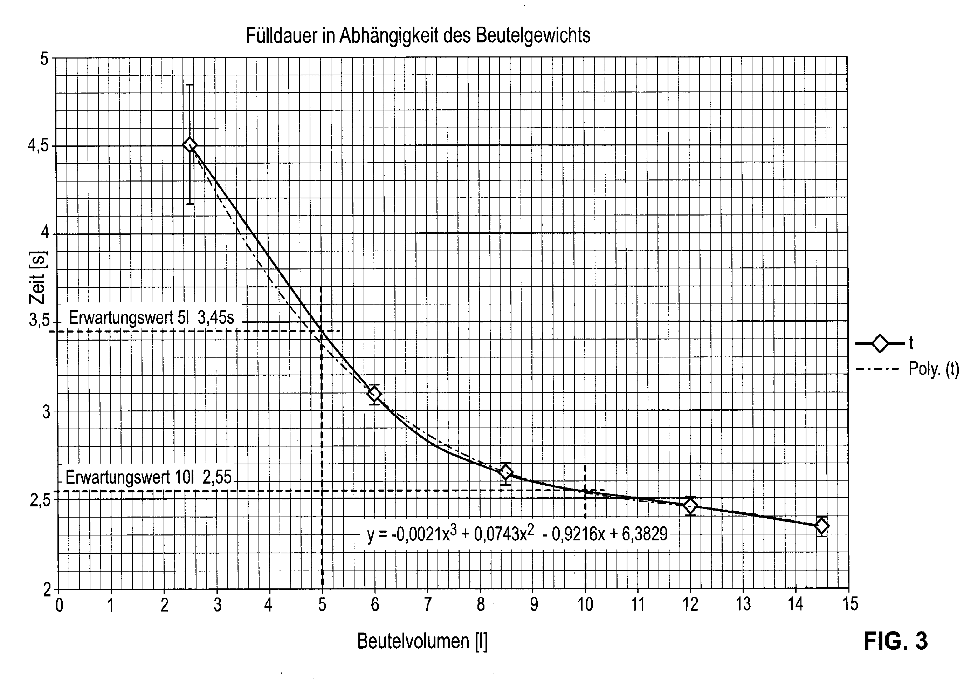

Figur 3 zeigt eine beispielhaft gezeigte Kennlinie, bei der auf der Abszisse die Gewichtskraft und auf der Ordinate die Öffnungsdauer der Ventile hinterlegt sein kann.

Die Erfindung kann vorsehen, dass in Abhängigkeit des auf der Waage aufgelegten Gewichts mittels einer Kennlinie eine Öffnungszeit der Ventile anhand der variablen Gewichtsänderung während des Fluidentnahme bis zum vollständigen Füllen der Patientenleitung ermittelt werden kann um einen luftfreien Zustand in der Patientenleitung zu gewährleisten.

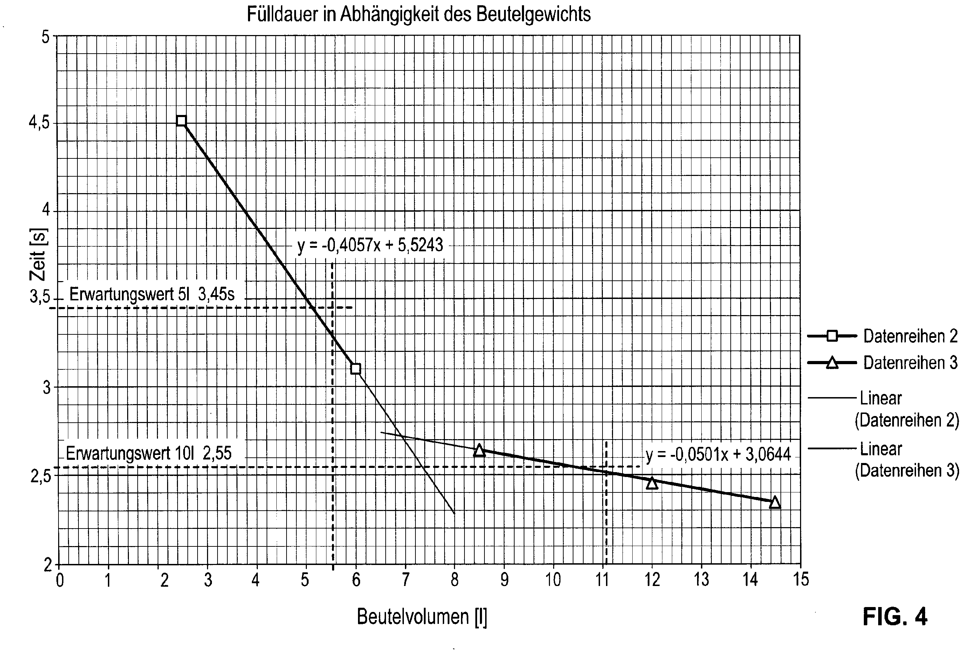

Figur 4 zeigt eine Kennlinie mit linearisierten Datenreihen.

Hierzu werden zunächst alle Ventile geöffnet (Beutel- und Drainageventile) um mögliche Partikel, die z.B. vom Öffnen der Brechkonen herrühren können, und Luft in den Drainagebeutel zu spülen. Im Anschluss daran wird der Patient aufgefordert, die Schlauchklemme zur Patientenleitung zu öffnen um diese zu spülen. Gleichzeitig kann mit der erfindungsgemäßen Idee eine anwenderbedingte Fehlbedienung erkannt werden.

Gerade bei Geräten, die ohne große technische Ausstattung betrieben werden, ist dies oft schwierig zu gewährleisten, da bestimmte Prozessabläufe in der Verantwortung des Anwenders / Patienten liegen. Insbesondere für die Anwendung im Heimbereich ist daher eine erhöhte Patientensicherheit wünschenswert. Mit der Erfindung lässt sich in einer weiteren Ausführungsform eine ordnungsgemäße Öffnung der Dialysatbeutelkonnektoren überprüfen. Dabei werden zunächst nur die beiden Schlauchventile (V1, V2) geöffnet um die Dialyseflüssigkeit bis zum Drainageventil strömen zu lassen. Das Drainageventil (V3) bleibt geschlossen.

Die Flüssigkeit verbleibt somit im Schlauchset. Es stellt sich ein neues statisches Gleichgewicht hinter der Waage ein. Somit lässt sich nun eine Gewichtsänderung bestimmen, da das abgeflossene Fluid den Drainagebeutel auf der Drainschale noch nicht erreicht hat, welche ebenfalls mit der einen Wägezelle in Verbindung steht. Daher kann über die Gewichtsänderung der Dialysatbeutel auf der Wägeschale auf einen stattgefundenen Fluidfluss geschlossen werden und somit ein Rückschluss erfolgen, ob die Beutelkonnektoren und /oder Schlauchklemmen geöffnet wurden bzw. eine Fehlbedingung vorliegt.

In einer weiteren Ausführungsform kann das Drainageventil V3 kurzzeitig geöffnet werden, bis ein Flüssigkeitsdurchfluss erkannt wird und bevor die Flüssigkeit die Drainageschale bzw. den Drainagebeutel erreicht hat. Wurde in einem ersten Schritt festgestellt, dass die Beutelkonnektoren und/oder die Schlauchklemmen ordnungsgemäß geöffnet wurden, kann in einem zweiten Schritt geprüft werden, ob die Schlauchklemme zum Drainagebeutel geöffnet ist. Hierzu werden die Schlauchventile V1 und/oder V2 als auch das Drainageventil V3 geöffnet. Lässt sich nun eine Gewichtsänderung zwischen der letzten Messung (Gewichtsänderung aufgrund von Fluidfluss in dem Schlauchsystem) und der Messung (Fluid vom Schlauchsystem in Drainagebeutel) erkennen, so lässt dies den Rückschluss zu, dass auch die Schlauchklemme zum Drainagebeutel geöffnet ist.

Denkbar ist es, an dem Drainageschlauch ein Flüssigkeitsreservoir anzubringen, um die Trübung des Drainfluids bestimmen zu können.

Nachteile bestehender Verfahren bzw. Vorrichtungen bestehen darin, dass der Patient den Spülvorgang überwachen muss, Ventile von Hand bedienen muss und schnell beim Schließen der Ventile reagieren muss. Damit sind die bekannten Verfahren bzw. Vorrichtungen fehleranfällig, verlangen einen höheren Patientenaufwand und bieten weniger Patientenkomfort.

Vorteile der Erfindung bestehen demgegenüber darin, dass ein Spülen automatisch durchgeführt werden kann und die Behandlung damit weniger fehleranfällig bspw. für das Einleiten von Luft, für nicht gebrochene Konen der Lösungsbeutel ist.

Ein Überfüllen der Patientenleitung und damit ein Auslaufen des Dialysats aus der gelochten Schutzkappe des Patientenkonnektors kann vermieden werden, wodurch eine Kontamination des Patientenkonnektors und eine Bauchfellentzündung vermieden werden.

Erfindungsgemäß kann die Gewichtsveränderung beim Starten des Priming des Tubing bzw. des Spülens erkannt werden und es kann dem Anwender unmittelbar gemeldet werden, wenn das Priming nicht gestartet werden kann

Erfindungsgemäß kann ferner erkannt werden, ob ein Füllen der Patientenleitung gestartet werden kann und es kann meldet gemeldet werden, wenn ein Füllen der Patientenleitung nicht möglich ist.

## Patentansprüche

1. Verfahren zum automatischen, gewichtsabhängigen Füllen eines Schlauchsystems, insbesondere für einen gravimetrischen Cycler für die Peritonealdialyse mit nur einer Waage (340), wobei das Schlauchsystem wenigstens drei Leitungsabschnitte zum Verbinden mit wenigstens einem Drainagebeutel, wenigstens einem Lösungsbeutel und einem Patienten umfasst, wobei wenigstens ein Beutelventil (V1, V2) und wenigstens ein Drainageventil (V3) geöffnet werden, **dadurch gekennzeichnet, dass** eine erste Öffnungsdauer der Ventile (V1,V2,V3) aus dem mittels der Waage gemessenen Gewicht des Lösungsbeutels und aus wenigstens einer Kennlinie abgeleitet wird.

2. Verfahren nach Anspruch 1, **dadurch gekennzeichnet, dass** mittels der Waage (340) erfasst wird, ob die Ventile (V1,V2,V3) geöffnet worden sind.

3. Verfahren nach Anspruch 1 oder 2, **dadurch gekennzeichnet, dass** eine Gewichtsabnahme des Lösungsbeutels während der Öffnung der Ventile (V1,V2,V3) mittels der Waage (340) erfasst wird und in Abhängigkeit von der erfassten Gewichtsabnahme die erste Öffnungsdauer modifiziert wird.

4. Verfahren nach Anspruch 1, 2 oder 3, **dadurch gekennzeichnet, dass** nach Schließen der Ventile (V1,V2,V3) eine Aufforderung an eine Bedienperson ergeht, eine Schlauchklemme zu öffnen und hierdurch Dialyseflüssigkeit in Richtung eines Konnektors (B,C, D, L) geleitet wird.

5. Verfahren nach einem der vorhergehenden Ansprüche, **dadurch gekennzeichnet, dass** mittels der Wage (340) erfasst wird, ob die Schlauchklemme geöffnet worden ist.

6. Verfahren nach einem der vorhergehenden Ansprüche, **dadurch gekennzeichnet, dass** wenigstens ein Schlauchventil sowie wenigstens ein Drainageventil (V3) geöffnet wird und dass aus einer Gewichtsänderung zwischen der Messung (Gewichtsänderung aufgrund von Fluidfluss in dem Schlauchsystem) und der Messung (Fluid vom Schlauchsystem in den Drainagebeutel) rückgeschlossen wird, dass die Schlauchklemme zum Drainagebeutel geöffnet ist.

7. Vorrichtung zum automatischen, gewichtsabhängigen Füllen eines Schlauchsystems nach einem der Ansprüche 1 bis 6, insbesondere für einen gravimetrischen Cycler für die Peritonealdialyse mit nur einer Waage, wobei das Schlauchsystem wenigstens drei Leitungsabschnitte zum Verbinden mit wenigstens einem Drainagebeutel, wenigstens einem Lösungsbeutel und einem Patienten umfasst, **dadurch gekennzeichnet, dass** die Vorrichtung eine Regelung/Steuerung umfasst, in der wenigstens eine Kennlinie hinterlegt ist, die Ventil-Öffnungsdauern in Abhängigkeit von dem Gewicht eines Lösungsbeutels angibt.

8. Vorrichtung nach Anspruch 7, **dadurch gekennzeichnet, dass** die Vorrichtung zum Öffnen von wenigstens einem Beutelventil (V1, V2) und/oder Drainageventil (V3) in Abhängigkeit von dem Gewicht des Lösungsbeutels eingerichtet ist.

9. Vorrichtung nach Anspruch 7 oder 8, **dadurch gekennzeichnet, dass** die Regelung/Steuerung dazu ausgelegt ist, anhand einer erfassten Gewichtsänderung das korrekte Öffnen des Beutelventils (V1 ,V2) und/oder Drainageventils (V3) zu verifizieren.

10. Vorrichtung nach Anspruch 7, 8 oder 9, **dadurch gekennzeichnet, dass** die Vorrichtung eine Ausgabeeinrichtung umfasst, die zum Ausgeben von Informationen an eine Bedienperson ausgebildet ist.

11. Vorrichtung nach einem der Ansprüche 7 bis 10, **dadurch gekennzeichnet, dass** die Regelung/Steuerung dazu ausgelegt ist, anhand einer erfassten Gewichtsänderung das korrekte Öffnen eines weiteren Ventils, insbesondere einer manuell bedienbaren Schlauchklemme zu verifizieren.

12. Peritonealdialysegerät mit wenigstens einer Vorrichtung gemäß einem der Ansprüche 7 bis 11.

## Claims

1. A method for the automatic, weight-dependent filling of a hose system, in particular for a gravimetric cycler for peritoneal dialysis, having only one scale (340), wherein the hose system comprises at least three line sections for connection to at least one drainage bag, to at least one solution bag and to a patient, wherein furthermore at least one bag valve (V1, V2) and at least one drainage valve (V3) are opened and a first opening duration of the valves (V1, V2, V3) is derived from the weight of the solution bag measured by means of the scale and from at least one characteristic.

2. A method in accordance with claim 1, **characterized in that** whether the valves (V1, V2, V3) have been opened is detected by means of the scale (340).

3. A method in accordance with claim 1 or claim 2, **characterized in that** a weight reduction of the solution bag during the opening of the valves (V1, V2, V3) is detected by means of the scale (340) and the first opening duration is modified in dependence on the detected weight reduction.

4. A method in accordance with claim 1, claim 2 or claim 3, **characterized in that**, after closing the valves (V1, V2, V3), a prompt is issued to an operator to open a hose clamp and **in that** dialysis fluid is hereby conducted in the direction of a connector (B, C, D, L).

5. A method in accordance with one of the preceding claims, **characterized in that** whether the hose clamp has been opened is detected by means of the scale (340).

6. A method in accordance with one of the preceding claims, **characterized in that** at least one hose valve as well as at least on drainage valve (V3) is opened; and **in that** a conclusion that the hose clamp to the drainage bag is open is drawn from a weight change between the measurement (weight change due to fluid flow in the hose system) and the measurement (fluid from the hose system into the drainage bag).

7. An apparatus for the automatic, weight-dependent filling of a hose system in accordance with one of the claims 1 to 6, in particular for a gravimetric cycler for peritoneal dialysis, having only one scale, wherein the hose system comprises at least three line sections for connection to at least one drainage bag, to at least one solution bag and to a patient, **characterized in that** the apparatus comprises a regulation/control in which at least one characteristic is stored which indicates valve opening durations in dependence on the weight of a solution bag.

8. An apparatus in accordance with claim 7, **characterized in that** the apparatus is configured for opening at least one bag valve (V1, V2) and/or drainage valve (V3) in dependence on the weight of the solution bag.

9. An apparatus in accordance with claim 7 or claim 8, **characterized in that** the regulation/control is configured to verify the correct opening of the bag valve (V1, V2) and/or drainage valve (V3) with reference to a detected weight change.

10. An apparatus in accordance with claim 7, claim 8 or claim 9, **characterized in that** the apparatus comprises an output device which is configured for outputting information to an operator.

11. An apparatus in accordance with one of the claims 7 to 10, **characterized in that** the regulation/control is configured to verify the correct opening of a further valve, in particular of a manually operable hose clamp, with reference to a detected weight change.

12. A peritoneal dialysis machine having at least one apparatus in accordance with one of the claims 7 to 11.

## Revendications

1. Procédé de remplissage automatique d'un système de tuyaux en fonction du poids, en particulier pour un cycleur gravimétrique pour la dialyse péritonéale, comportant seulement une balance (340), le système de tuyaux comprenant au moins trois parties de tubulure pour la liaison à au moins une poche de drainage, au moins une poche de solution et un patient, au moins une valve de poche (V1, V2) et au moins une valve de drainage (V3) étant ouvertes, **caractérisé en ce qu'**une première durée d'ouverture des valves (V1, V2, V3) est dérivée du poids de la poche de solution mesuré au moyen de la balance et d'au moins une courbe caractéristique.

2. Procédé selon la revendication 1, **caractérisé en ce qu'**il est détecté au moyen de la balance (340) si les valves (V1, V2, V3) ont été ouvertes.

3. Procédé selon la revendication 1 ou 2, **caractérisé en ce qu'**une diminution de poids de la poche de solution est détectée pendant l'ouverture des valves (V1, V2, V3) au moyen de la balance (340) et la première durée d'ouverture est modifiée en fonction de la diminution de poids détectée.

4. Procédé selon la revendication 1, 2 ou 3, **caractérisé en ce que**, après la fermeture des valves (V1, V2, V3), une demande est envoyée à une personne opératrice, afin qu'elle ouvre une pince pour tuyau et, ainsi, un liquide de dialyse est guidé en direction d'un connecteur (B, C, D, L).

5. Procédé selon l'une des revendications précédentes, **caractérisé en ce qu'**il est détecté au moyen de la balance (340) si la pince pour tuyau a été ouverte.

6. Procédé selon l'une des revendications précédentes, **caractérisé en ce qu'**au moins une valve de tuyau ainsi qu'au moins une valve de drainage (V3) sont ouvertes et que, à partir d'une modification de poids entre la mesure (modification de poids en raison de l'écoulement de fluide dans le système de tuyaux) et la mesure (fluide du système de tuyaux vers la poche de drainage), il est déduit que la pince pour tuyau vers la poche de drainage est ouverte.

7. Dispositif de remplissage automatique d'un système de tuyaux en fonction du poids selon l'une des revendications 1 à 6, en particulier pour un cycleur gravimétrique pour la dialyse péritonéale comportant seulement une balance, le système de tuyaux comprenant au moins trois parties de tubulure pour la liaison à au moins une poche de drainage, au moins une poche de solution et un patient, **caractérisé en ce que** le dispositif comprend un dispositif de régulation/commande, dans lequel est enregistrée au moins une courbe caractéristique, qui indique des durées d'ouverture de valve en fonction du poids d'une poche de solution.

8. Dispositif selon la revendication 7, **caractérisé en ce que** le dispositif est conçu pour ouvrir au moins une valve de poche (V1, V2) et/ou valve de drainage (V3) en fonction du poids de la poche de solution.

9. Dispositif selon la revendication 7 ou 8, **caractérisé en ce que** le dispositif de régulation/commande est conçu pour vérifier, à l'aide d'une modification de poids détectée, l'ouverture correcte de la valve de poche (V1, V2) et/ou de la valve de drainage (V3).

10. Dispositif selon la revendication 7, 8 ou 9, **caractérisé en ce que** le dispositif comprend un moyen de sortie, qui est conçu pour fournir en sortie des informations à une personne opératrice.

11. Dispositif selon l'une des revendications 7 à 10, **caractérisé en ce que** le dispositif de régulation/commande est conçu pour vérifier, à l'aide d'une modification de poids détectée, l'ouverture correcte d'une autre valve, en particulier d'une pince pour tuyau actionnable manuellement.

12. Appareil de dialyse péritonéale comportant au moins un dispositif selon l'une des revendications 7 à 11.
